# EUROPEAN PATENT APPLICATION

(11) **EP 0 634 152 A1**
(43) Date of publication of application: **18.01.1995**
(21) Application number: 94109185.2
(22) Date of filing: 15.06.1994
(51) Int. Cl.: A61F 2/06

(54) **Biodegradable urethal stent**

(30) Priority: 15.06.1993 FI 932752; 10.05.1994 FI 942170
(71) Applicant: Viherkoski, Esa, SF-00570 Helsinki (FI)
(72) Inventor: Viherkoski, Esa, SF-00570 Helsinki (FI)
(74) Representative: Grünecker, Kinkeldey, Stockmair & Schwanhäusser Anwaltssozietät

(57) **Abstract**

The invention concerns a tubular device for insertion in the urethra after transurethral laser irradiation procedure for temporarily keeping the urethra patent, said device consisting of a material which is biodegradable and/or dissolving in the organism. The device is substantially shorter than the urethra so that it can be totally disposed in the urethra.

## Description

The present invention concerns a tubular device intended to be installed in the urethra after transurethral laser irradiation intervention for the purpose of temporarily keeping the urethra patent. The invention also concerns use of said device in a transurethral laser irradiation therapy procedure.

Various urological disorders are highly common. In men, urological problems are most often connected with the prostatic gland.

Prostatic complaints are usually a problem of aging men. A highly typical benign prostatic disorder, benign prostatic hyperplasia (BPH) is found in more than 50% of men over 50. BPH causes various problems interfering with the normal function of the urinary passages. In the U.S.A., for instance, about 400,000 BPH cases requiring therapy are detected annually. Therapy is implemented e.g. by reducing the prostate using transurethral resection of the prostate (TURP). Laser irradiation is a commonly accepted therapeutic procedure serving as one of the forms of therapy applied in various urological diseases and conditions. Lately, transurethral laser irradiation has also been adopted as an alternative therapy in cases of BPH e.g. to replace the surgical TURP method. Laser irradiation has also been applied in combination with said surgical procedure.

In the transurethral laser therapy procedure for BPH the hyperplastic prostate tissue is reduced by destroying prostate tissue surrounding the urethra with the aid of heat generated at laser irradiation. The destroyed necrotic tissue becomes detached and departs from around the urethra within about 3-4 weeks.

The advantage of the transurethral laser irradiation procedure e.g. over the surgical TURP method is that in the laser irradiation procedure haemorrhage at the object thus treated is significantly less, and the procedure is swift in implementation, whereby anaesthetizing the patient is not indispensably necessary and the therapy can be policlinically undertaken, implying lowered treatment costs in view of the patient as well as the unit giving the therapy. Furthermore, the risk of complications following on laser irradiation is markedly lower than that after surgical intervention.

Said laser irradiation procedure is however embarrassed by the problem that the living tissue surrounding the prostatic tissue which has been destroyed by irradiation reacts owing to the thermal effect that has been produced, whereby fluid accumulates in the tissue and the tissue swells powerfully. The strongly oedematous tissue compresses the urethra, choking it, and for this reason it is common practice after the intervention to install a catheter to keep the urethra patent for the duration of swelling. Removal or exchange of the catheter usually requires an extra visit to the outpatient clinic. Furthermore, wearing a catheter may be uncomfortable and cumbersome from the patient's point of view.

The object of the present invention is to eliminate the drawbacks mentioned.

The object of the invention is specifically to disclose a novel device serving to prevent occlusion of the urethra caused by the swelling subsequent to transurethral laser irradiation, which device need not be removed after it has once been introduced in the urethra and which is completely unnoticeable from the patient's point of view.

The object of the invention is further to disclose a novel transurethral laser irradiation therapy procedure thanks to which the patient is spared later visits to the outpatient clinic which have been required when using catheters of prior art.

Regarding the features characterizing the invention reference is made to the claims.

The invention is appropriate to be used particularly in connection with urological therapeutic procedures. The invention discloses in particular a tubular device consisting of material which is biodegradable and/or dissolving in the human organism, intended to be inserted in the urethra subsequent to a transurethral laser irradiation intervention for temporarily keeping the urethra patent. Furthermore, since the device of the invention need not be removed from the urethra later and, therefore, features conducive to easier removal need not be considered as regards the device, the device can be arranged to have a length substantially less than that of the urethra so that it can be totally inserted in the urethra. Thus the device of the invention is, for this reason too, less noticeable and cumbersome from the patient's point of view compared with catheters of prior art.

The device of the invention is tubular. The cross section of the device may be circular, elliptical or any other shape, provided that the shape allows unobstructed passage of urine through the tube and enables the device to be inserted in the urethra.

The length of the tubular device is selected to be consistent with the length of urethra which should be kept patent, and it may vary e.g. from a few millimetres to several centimetres.

The diameter of the device is consistent with previous practice known in the art. The retention of the device at the point where it has been installed can be influenced by means of its diameter.

The wall of the tubular device of the invention may be smooth or porous. Furthermore, the device may be a tube with reticular wall, a tubular helix or equivalent so that when inserted in the urethra it will retain its substantially tubular shape through a predetermined period of time.

According to an advantageous embodiment, the device of the invention is mainly tubular and in its wall apertures have been provided, whereby after the device has been installed in the urethra tissue destroyed by laser irradiation can escape from the point of treatment through the apertures in the wall of the device. The apertures may be regular, e.g. with circular, rectangular or rhombic shape, or irregular. Furthermore, the apertures may be disposed in the wall of the device with regular or irregular spacing. The size of the apertures is not limited. The apertures are suitably so selected that tissue which has been destroyed in the laser irradiation treatment can enter the tubular device through said apertures from the tissue surrounding the device and can be carried away from the device e.g. along with urine, and so that the tubular device will retain its substantially rigid shape maintaining the urethra patent.

The device may consist of one or several structural elements, possibly comprising e.g. layers, coatings or reinforcing wires which have an influence on the supporting and/or disintegrating properties of the device. The surface configuration of the wall may also be used to exert an effect on retention of the device.

If desired, one end of the device of the invention may be shaped so as to facilitate its insertion.

The material used for the device of the invention may be any material which is biodegraded and/or dissolved in the organism, implying in this context that when it has fulfilled its function the material will be eliminated through cellular vital functions and/or by dissolving. The material may be decomposed e.g. owing to mucous membrane contact and/or by effect of urine. As an example may be mentioned polymer materials used in elements intended for surgical joining of tissues and decomposing by themselves in the organism, such as polyglycolide (PGA), polylactide (PLA), glycolide/lactide mixed polymers, glycolide/trimethylene carbonate mixed polymers, polydioxanone, polyhydroxybutyrate, polyhydroxypropionate, mixtures and copolymers of said polymers, chitin polymers, polyester amides, glycolesters, etc.

Devices according to the invention are made e.g. by utilizing techniques known from the context of biodegradable and/or dissolving devices. The desired urethra patency maintaining time/decomposition time is regulated in commonly known manner e.g. with the aid of the material used, the structure of the tube, the material quantity used for the tube, etc. Regarding biodegradable and/or dissolving materials, and technology of prior art, reference is made e.g. to the patents FI 83477, FI 84137. If desired, additives known in the art may be included in the polymer material used, to modify the properties of the material.

On being inserted in the urethra, the device of the invention substantially retains its shape during at least about a few days up to 3 weeks, more advantageously several months, e.g. 3-12, such as 6-7 months. A device of prolonged permanence has the advantage that the urethral functions have time to be properly normalized.

According to an embodiment, the device of the invention contains pharmaceutically active substance, which has been incorporated in the biodegradable material of the device so as to be released at the point of insertion of the device into surrounding tissue, e.g. within a given period of time.

The device is suitable with particular advantage to be used to keep the urethra temporarily patent after transurethral laser irradiation of the prostate, the device being inserted, possibly with the aid of a scope means or another introductor, in the urethra adjacent to the treated prostate, where it renders support to the urethra so that the living prostatic tissue which has swollen as a result of the intervention cannot collapse the urethra.

It is thus understood that the invention furthermore concerns a therapeutic procedure, the patient being subjected to a transurethral laser irradiation intervention, whereupon a tubular device according to the invention is inserted entirely in the urethra, in register with the laser-irradiated region, for temporarily keeping the urethra patent.

A device according to the invention can naturally equally be introduced in other body passages or ducts after laser irradiation interventions, or other treatments, performed from them, in order to keep the respective passages or ducts patent e.g. for the duration of swelling, or for another reason.

The invention is described in greater detail in the following with the aid of embodiment examples, referring to the drawings, wherein
Fig. 1 presents a tubular device according to the invention,
Fig. 2 presents another tubular device according to the invention,
Fig. 3 presents a third tubular device according to the invention, and
Figs 4a-c present various embodiments of a fourth tubular device according to the invention.

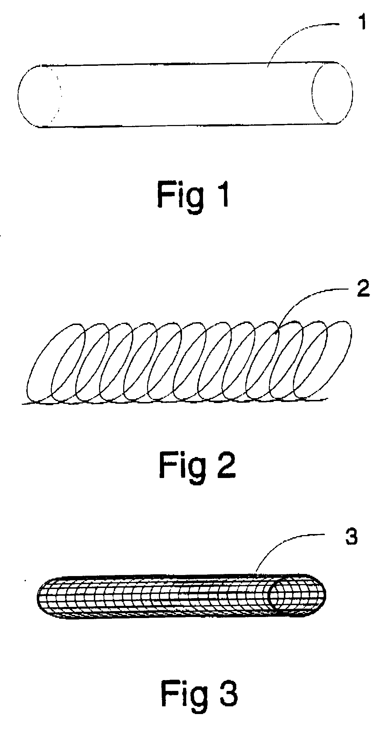

In Figs 1-3 are presented some alternatively formed devices according to the invention, viz., a smooth tube 1, a tube with reticular wall 3, and a tubular helix 2. The cross section of tubes 1 and 3 is circular and that of tube 2 has elliptic shape. The length of the tube 1,2,3 is about 1-6 cm, e.g. 3-5 cm, and the diameter of tubes 1 and 3 and the diameter of tube 2 at the widest point of the ellipse is about 4-6 mm. Each tube is intended to be placed in the urethra adjacent to the prostate. The tube material and the wall thickness of the tube are so selected that the tube 1,2,3 substantially retains its shape for at least about 1-2 weeks.

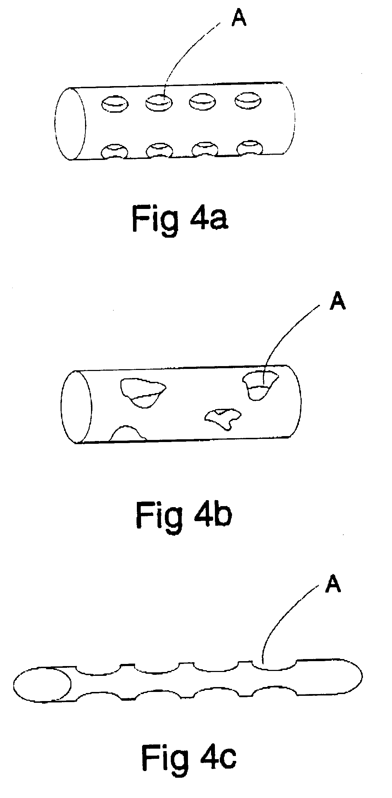

In Fig. 4 are depicted mainly tubular devices 4a, 4b and 4c according to an advantageous embodiment of the invention. In the wall of the device 4a round apertures A have been provided with substantially regular spacingnd in the wall of device 4b irregular apertures A have been provided with irregular spacing; devices 4a and 4b have circular cross section. The cross section of device 4c is elliptical, and in its wall apertures A have been provided on opposite sides. The walls 4 of devices 4a-4c consist of mainly unbroken material except for said apertures A. The diameters of devices 4a, 4b and 4c are consistent with what has been said above. Furthermore, their material and wall thickness have been so selected that they will substantially retain their shape through 3-12 months, e.g. 6-7 months.

The apertures A provided in the device may be equal or unequal in size. The size of the apertures A is selected to be such that destroyed tissue can pass through them.

The devices of the invention may contain pharmaceutically active substance, such as antibiotic, cytostat, anaesthetic agent or equivalent, so that the active substance can become released from the device at its point of installation, e.g. in the course of a given period of time.

### Example 1: An advantageous application according to the invention

A BHP patient is subjected to transurethral prostate laser irradiation. Laser irradiation is performed with a laser apparatus of Nd:YAG type (YAGUAR, Model 3580, Lasermatic) using a scope tube as aid. The optic cable disposed in the scope tube is rotated so as to achieve focussing of the irradiation on four quadrants of the prostate surrounding the urethra. The laser irradiation is produced with power about 40-60 W, the irradiation time of each one quadrant being about 60 s. The procedure is monitored with the aid of a camera mounted on the scope tube. Monitoring may equally take place in another way, e.g. with the aid of monitoring based on ultrasonics. The irradiated prostatic tissue is destroyed by coagulation, among other modes.

The swelling of prostatic tissue attendant on this treatment acts on the urethra adjacent to the prostate on a length about 1-2 cm, and therefore subsequent to the treatment a device according to the invention, its length equalling said distance or slightly more, is introduced in the urethra using the scope tube for introductor, to extend over the whole length of said interval.

The device thus installed remains in the urethra, being unnoticeable and significantly less cumbersome compared with catheters of prior art. Moreover, the actions required in connection with laser irradiation suffice for accomplishing the whole treatment procedure, because the device of the invention need not be removed at any later time.

The device of the invention substantially retains its shape throughout the duration of objectionable swelling, that is, for at least about 1-2 weeks, and advantageously for several months, e.g. 6-7 months, rendering feasible normal functions of the urethra, whereafter the tube is gradually decomposed of itself by effect of mucous membrane contact and/or urine.

Devices according to the invention with apertures, 4a, 4b and 4c, have in practice been found to be significantly advantageous in that the destroyed tissue can escape with ease from the point of treatment through the apertures of the device. Furthermore, the presence of apertures makes the tube flexible and thereby unnoticeable to the user, i.e., user-friendly.

The embodiment example is merely intended to illustrate the invention, without confining it.

## Claims

1. A tubular device for insertion in the urethra after transurethral laser irradiation procedure for temporarily keeping the urethra patent, characterized in that the device consists of material which is biodegradable and/or dissolving in the organism, and the device is substantially shorter than the urethra so that it can be totally disposed in the urethra.

2. Device according to claim 1, characterized in that the cross section of the device has substantially circular or elliptic shape.

3. Device according to claim 1, characterized in that in the wall of the tubular device apertures have been provided.

4. Device according to any one of claims 1-3, characterized in that the device substantially retains its shape in the urethra for at least about 1-3 weeks, e.g. at least one month, advantageously 3-12 months.

5. Device according to any one of claims 1-4, characterized in that the device consists of biodegradable and/or dissolving polymer material, such as polyglycolide (PGA), polylactide (PLA), glycolide/lactide mixed polymers, glycolide/trimethylene carbonate mixed polymers, polydioxanone, polyhydroxybutyrate, polyhydroxypropionate, mixtures and copolymers of said polymers, chitin polymers, polyester amides or glycolesters.

6. Device according to any one of claims 1-5, characterized in that the transurethral laser irradiation procedure is a transurethral prostate laser irradiation procedure.

7. A therapeutic procedure, the patient being subjected to transurethral laser irradiation, whereafter in the urethra is inserted a tubular device for temporarily keeping the urethra patent, characterized in that in the urethra is inserted a device consisting of material which is biodegraded and/or dissolved in the organism, and which is substantially shorter than the urethra so that it becomes disposed totally within the urethra.

8. Procedure according to claim 7, characterized in that the patient is subjected to transurethral laser irradiation of the prostate and in the urethra is inserted a tubular device which in the urethra substantially retains its shape at least about a few days to three weeks, e.g. at least one month, advantageously 3-12 months.

9. Use of a tubular device according to claim 1 after a transurethral laser irradiation procedure for temporarily keeping the urethra patent.

10. Use of a tubular device according to claim 9 in connection with transurethral prostate laser irradiation.
